(19) Europäisches Patentamt
European Patent Office
Office européen des brevets

(11) Veröffentlichungsnummer: **0 485 381 B1**

(12) **EUROPÄISCHE PATENTSCHRIFT**

(45) Veröffentlichungstag der Patentschrift: **15.06.94**

(51) Int. Cl.$^5$: **A61K 31/365**

(21) Anmeldenummer: **90903011.6**

(22) Anmeldetag: **16.02.90**

(86) Internationale Anmeldenummer:
**PCT/EP90/00251**

(87) Internationale Veröffentlichungsnummer:
**WO 90/09790 (07.09.90 90/21)**

(54) **VERWENDUNG VON MACROLACTONEN ALS ANTIALLERGICA.**

(30) Priorität: **28.02.89 DE 3906214**

(43) Veröffentlichungstag der Anmeldung:
**20.05.92 Patentblatt 92/21**

(45) Bekanntmachung des Hinweises auf die Patenterteilung:
**15.06.94 Patentblatt 94/24**

(84) Benannte Vertragsstaaten:
**AT BE CH DE DK ES FR GB IT LI LU NL SE**

(56) Entgegenhaltungen:

**The Journal of Antibiotics, volume 39, no. 5 May 1986. D. Rodphaya et al.: pages 629-635**

**Patent Abstracts of Japan, volume 12, no. 191(C-501) (3038), page 54 C 501 & JP A 62294676(TAKEDA CHEM.IND. LTD) 22 December 1987**

**Agric. Biol. Chem., volume 49, no. 3, 1985, A. Hirota et al, pp.731-735**

(73) Patentinhaber: **BOEHRINGER MANNHEIM GMBH**

**D-68298 Mannheim(DE)**

(72) Erfinder: **HANSSKE, Fritz**
**Jahnstr. 32 b**
**D-6945 Hirschberg(DE)**
Erfinder: **WILHELMS, Otto-Henning**
**Odenwaldstr. 25/2**
**D-6941 Weinheim-Rittenweier(DE)**
Erfinder: **ANKE, Heidrun**
**Theodor-Heuss-Str. 17**
**D-6750 Kaiserslautern(DE)**

(74) Vertreter: **Schroth, Helmut, Dr. et al**
**c/o Boehringer Mannheim GmbH,**
**Patentabteilung,**
**Sandhoferstrasse 116**
**D-68298 Mannheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von Macrolactonen der allgemeinen Formel

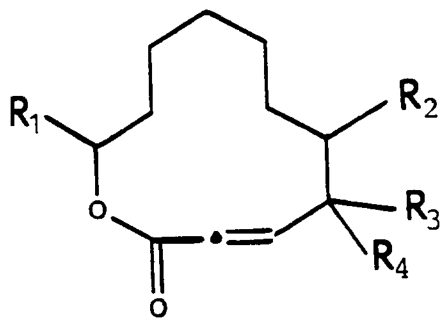

I

worin
$R_1$ ein Wasserstoffatom oder einen $C_1$-$C_4$-Alkyl-Rest,
$R_2$ ein Wasserstoffatom, einen Hydroxyl- oder einen Acetoxyrest
$R_3$, $R_4$ jeweils unabhängig voneinander ein Wasserstoffatom, einen Hydroxyl- oder ein Acetoxyrest oder zusammen ein Sauerstoffatom
und
- ● = ein cis- oder trans-konfiguriertes Kohlenstoffzentrum darstellen zur Herstellung eines Arzneimittels für die Behandlung allergischer Beschwerden, insbesondere zur Hemmung der Aktivierung proinflammatorisch wirkender Leukozyten wie z. B. der Antigeninduzierter Degranulation peripherer humaner Leucozyten.

Sämtliche von der allgemeinen Formel 1 umfaßten Verbindungen sind bekannt und beschrieben bzw. können in analoger Weise aus bekannten Verbindungen hergestellt werden.

So wurden die Cladospolide A und B aus Cladosporium cladosporioides isoliert (A. HIROTA et al., Agric. Biol. Chem., 49 ((1985) 731). Die absolute Stereochemie an den drei asymmetrischen Zentren wurde durch einschlägige NMR-Methoden und Röntgenstrukturanalyse bestimmt (H. HIROTA et al., Bull. Chem. Soc. Jpn., 58 (1985) 2147; A. HIROTA et al., Agric. Biol. Chem., 49 ((1985) 903). Die Totalsynthese von Cladospolid A wurde kürzlich beschrieben (K. MORI et al., Liebigs Ann. Chemie, ((1987) 863).

Die Diacetate der Cladospolide A und B werden in an sich bekannter Weise durch Acetylierung in Pyridin mit Acetanhydrid erhalten.

Die Patulolide A, B und C wurden aus Penicillium urticae-Mutanten S 11 (ATCC 48165) oder S 11 R 59 (IFO 31725) isoliert (D. RODPHAYA et al., J. Antibiotics, 39 (1986) 629; J. SEKIGUCHI et al, Tetrahedron Letters, 26, ((1985) 2341).

Die Biosynthese dieser Verbindungen wurde kürzlich aufgeklärt (D. RODPHAYA et al., J. Antibiotics, 41 ((1988) 1649).

Die Totalsynthesen wurden ebenfalls kürzlich beschrieben (K. MORI et al., Liebigs Ann. Chem. ((1988) 13).

Die bisher bekannte biologische Aktivität der Macrolactone gemäß Anspruch 2 ist auf folgende Bereiche beschränkt:
Cladospolid A wirkt schwach inhibierend auf das Wurzelwachstum von Salatkeimen (Lactuca sativa) während Cladospolid B im gleichen Testsystem fördernd wirkt (A. HIROTA et al., Agric. Biol. Chem., 49 (-(1985) 731). Die Hydrierungsprodukte der Cladospolide A und B wurden ebenfalls beschrieben. Sie besitzen keine biologische Aktivität im obigen Sinne (A. HIROTA et al., Agric. Biol. Chem., 49 ((1985) 731).

Die Patulolide A, B und C wurden als schwache fungistatische und antibakterielle Wirkstoffe aber auch als Pflanzenwachstumshemmstoffe beschrieben (D. RODPHAYA et al., J. Antibiotics, 39 (1986) 629; JP-222192, 4.10.1985, Takeda Chemical IND. K.K.).

Es konnte nun überraschend in einfachen in vitro-Screenings-systemen gefunden werden, daß die erfindungsgemäßen Verbindungen die Aktivierung von Leukozyten zur Mediatorfreisetzung hemmen und darüber hinaus auch eine Spasmus-hemmende, antiallergische Aktivität aufweisen. Diese Wirkung wurde nachgewiesen in dem Prüfverfahren für Antiallergica, der Antigen-bedingte Konstriktion von passiv sensibilisierten Meerschweinchen-Lungenparenchymstreifen.

Gegenstand der vorligenden Erfindung ist deshalb die Verwendung von Macrolactonen der allgemeinen Formel I:

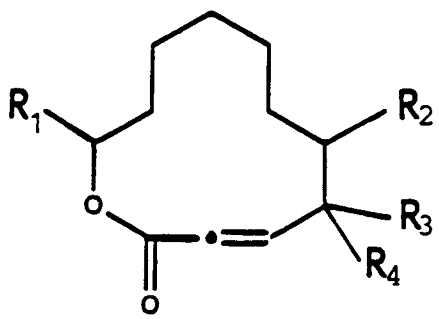

I

worin
$R_1$ ein Wasserstoffatom oder einen $C_1$-$C_4$-Alkyl-Rest,
$R_2$ ein Wasserstoffatom, einen Hydroxyl- oder einen Acetoxyrest
$R_3$, $R_4$ jeweils unabhängig von einander ein Wasserstoffatom, einen Hydroxyl- oder ein Acetoxyrest oder zusammen ein Sauerstoffatom
und
- • = ein cis- oder trans-konfiguriertes Kohlenstoffzentrum darstellen zur Behandlung von allergischen Erkrankungen.

Darin wird unter $C_1$-$C_4$-Alkyl ein Methyl-, Ethyl-, Propyl- oder Butylrest verstanden, vorzugsweise Methyl.

Ganz besonders bevorzugt sind die Verbindungen, in denen $R_1$ = Methyl und $R_2$ = Wasserstoff und $R_3$ und $R_4$ zusammen ein Sauerstoffatom darstellen; die Doppelbindung besitzt cis- oder trans-Substitution.

Für den Nachweis der antiallergischen Wirkung wurde die Hemmung der durch Anti-IgE-Antikörper induzierten Proteinasefreisetzung aus peripheren Human-Leukozyten (Wilhelmset al. XII. Congress Europ. Acad. Allergology Clin. Immunology, Rom, Sept. 25. - 30., 1983 sowie DE-A1-34 46 714) oder der Histaminfreisetzung in einem analogen Verfahren nach einer Weiterentwicklung der Methode von R.P. Siraganian und W.A. Hook (Manual of Clinical Immunology, 2. Auflage 1980, Herausg. N.R. Rose und H. Friedman, Am. Soc. for Microbiology, Seiten 808 bis 821) gearbeitet.

Beschreibung des Testsystems für die Prüfung auf Mediatorfreisetzungshemmung aus Human-Leukozyten in vitro

Prinzip:

An isolierten Human-Leukozyten wird durch Stimulation mit Anti-IgE-Antikörper die Freisetzung von Proteinase ausgelöst (IgE-vermittelte Mediatorfreisetzung aus basophilen Granulozyten). Durch Substrat-Zugabe (Chromozym TH) und photometrischer Messung wird die Intensität dieser Freisetzungs-Reaktion bestimmt, indem die Farbstoff-Intensität der Proben ± Hemmstoffzusatz verglichen werden.

Zellmaterial:

Buffy-coat-Zellen = konzentrierte Zellsuspension aus Blutkonserven

In der nachfolgenden Tabelle I sind die Hemmwerte der Anti-IgE-induzierten Freisetzung von Histamin und Proteinase aus peripheren humanen Leukozyten wiedergegeben. Als Vergleichssubstanz wurde der bekannte Degranulationsinhibitor Theopyllin herangezogen.

**Tabelle I**

| Substanz | Konz. /ug/ml | % Hemung Histamin | Proteinase |
|---|---|---|---|
| **Cladospolid A** | 10<br>2 | 44<br>8 | 46<br>5 |
| **Diacetylcladospolid A*)** | 10<br>2 | 92<br>17 | 96<br>14 |
| **Patulolid A** | 10<br>2 | 100<br>44 | 99<br>46 |
| **Patulolid B** | 10<br>2 | 100<br>57 | 99<br>63 |
| **Theophyllin** | 90 | 72 | 70 |

*)
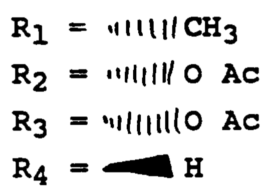

Als weiterer Nachweis für die antiallergische Wirkung wurde die Hemmung der Antigen-bedingten Konstriktion von passiv sensibilisierten Meerschweinchen-Lungenparenchymstreifen in vitro untersucht.

<u>Hemmung der Antigen-bedingten Konstriktion von passiv sensibilisierten Meerschweinchen - Lungenparenchym-Streifen in vitro (Organbad)</u>

Für die in vitro Untersuchung der erfindungsgemäßen Verbindungen wurde die Hemmung der Antigen - bedingten Konstriktion an passiv sensibilisierten Meerschweinchen-Lungenparenchym-Streifen gemessen, wie nachfolgend beschrieben.

Pirbright-White Meerschweinchen wurden betäubt durch Genickschlag und entblutet. Die Lungen wurden in situ mit Krebs-Puffer, pH 7.4, weitgehend blutfrei gespült.

Anschließend wurde die Lunge entnommen, in Streifen geschnitten (ca. 20 x 4 x 4 mm), und die Streifen für 1 Stunde bei Raumtemperatur mit einer 1:50-Verdünnung eines homologen Anti-Ov-Albumin-Antiserums passiv sensibilisiert und dann mit Krebs-Puffer 1 x gewaschen.

Das Antiserum war vorher nach DAVIES G.E., T.P. Johnstone (Quantitative studies on anaphylaxis in guinea-pigs passively sensitized with homologous antibody; Inter.Arch. <u>41</u>, 648 - 654 (1971)) in Meerschweinchen des gleichen Stammes erzeugt worden, durch wiederholte Injektion von Ov-Albumin (2 x kristallisiert) unter Zusatz von komplettem Freund'schem Adjuvans.

Bis zu seiner Verwendung wurde das Antiserum bei -18 °C unverdünnt gelagert.

Anschließend wurden die Lungenstreifen einzeln in 10 ml-Wasserbädern mit einer Vorspannung von 1.2 g an einem isometrischen Meßaufnehmer aufgehängt.

Danach wurden die Wasserbäder mit Krebs-Puffer gefüllt und kontinuierlich bei 37 °C mit $O_2$ (95%) und $CO_2$ (5%) begast.

Über einen Verstärker wurden die Konstriktionen der Lungenstreifen auf einem Schreiben aufgezeichnet.

Nach 30-minütiger Eingewöhnungsphase wurden Histamin-Kontrollspasmen zur Erkennung der Reaktionsfähigkeit der Organstücke erzeugt, gewaschen, anschließend für 20 Minuten die Prüfsubstanz bei 37 °C vorinkubiert und danach die Ov-Albumin-bedingte Konstriktion ausgelöst.

Die Hemmwirkungen der erfindungsgemäßen Verbindungen wurden als prozentuale Verringerung der Konstriktionsamplitude der "Proben mit Prüfsubstanz" im Verhältnis zu den "unbehandelten Kontrollkonstriktionen" ausgedrückt.

Die Ergebnisse sind in Tabelle II wiedergegeben.

Passiv sensibilisierter Meerschweinchen-Lungenparenchym-Streifen

**Hemmung der Antigen - bedingten Konstriktion durch potentielle Mediator - Freisetzungshemmer**

**( Organbad )**

**Spasmogene und Konzentrationen:**

| | | |
|---|---|---|
| A = Histamin | 1.0 | [mcg/ml] |
| B = Ov - Albumin | 0.1 | " |
| C = Glucose | 0.16 | [%] |
| D = Ov - Albumin | 1.0 | [mcg/ml] |

**n = Anzahl der Versuche** **R = Relaxation**

| Substanz | A | B | C | D | n |
|---|---|---|---|---|---|
| Diacetlycladospolid A 40.00 mcg/ml | 6 | 44 | 45 | 46 | 3 |
| Patulolid B 40.00 mcg/ml | 3 | 95 | 93 | 93 | 3 |
| Patulolid B 10.00 mcg/ml | 5 | 54 | 47 | 51 | 3 |
| Cladospolid A 40.00 mcg/ml | 16 | 34 | 33 | 24 | 3 |
| Cladospolid A 10.00 mcg/ml | -5 | -50 | -85 | -76 | 1 |
| Patulolid A 40.00 mcg/ml | 21 | 51 | 53 | 30 | 3 |
| Patulolid A 10.00 mcg/ml | 23 | 35 | 35 | 35 | 1 |
| Patulolid C 40.00 mcg/ml | -24 | -40 | -36 | -33 | 3 R |
| Patulolid C 10.00 mcg/ml | -38 | -35 | -98 | -26 | 3 |

Die erfindungsgemäß verwendeten Verbindungen der allgemeinen Formel I können in den üblichen, insbesondere für Antiallergica geeigneten pharmazeutischen Zubereitungen und Darreichungsformen verwendet werden, z.B. als Tabletten, Dragees, Suppositorien, Injektionslösungen, Säfte, Inhalationssprays usw.. Die Arzneimittel enthalten den Wirkstoff vorzugsweise zusammen mit üblichen pharmazeutischen Träger- und Verdünnungsmitteln, gegebenenfalls auch in Kombination mit anderen Wirkstoffen, wie z.B. weiteren Antiallergica, oder für die Allergietherapie weiteren geeigneten Wirkstoffen, wie z.B. Bronchorelaxantien, Anticholinergika, ß-Stimulantien, Mucolytica, antiinflammatorisch und fiebersenkden wirkenden Mitteln, Vitaminen, usw.. Die tägliche Verabreichungsdosis richtet sich insbesondere nach der Art und Schwere der Erkrankung; sie beträgt beim erwachsenen Menschen in der Regel 0,01 bis 100 mg Wirksubstanz, vorzugsweise 0,1 bis 10 mg.

Beispiel:

Diacetylcladospolid A

50 mg (0.22 mmol) Cladospolid A werden in 1 ml Pyridin mit 225 mg (2.2 mmol) Acetanhydrid bei Raumtemperatur 16 Std. gerührt. Nach standardmäßiger Aufarbeitung und Chromatographie an Kieselgel werden 59 mg chromatographisch reine Titelverbindung isoliert. Umkristallisation aus Hexan.
Smp. 74 °C.
$R_f$ (Sil, $CHCl_3$/MeOH = 9:1): 0.34, Cladospolid A; 0.68, Diacetylcladospolid A

**Patentansprüche**

1. Verwendung von Macrolactonen der allgemeinen Formel I:

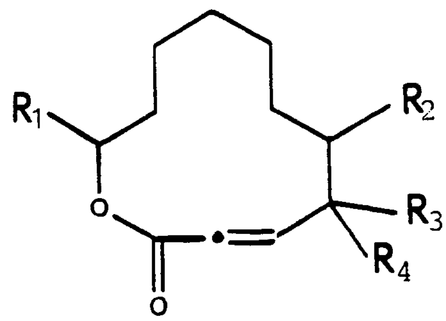

I

worin
$R_1$ ein Wasserstoffatom oder einen $C_1$-$C_4$-Alkyl-Rest,
$R_2$ ein Wasserstoffatom, einen Hydroxyl- oder einen Acetoxyrest
$R_3$, $R_4$ jeweils unabhängig von einander ein Wasserstoffatom, einen Hydroxyl- oder ein Acetoxyrest oder zusammen ein Sauerstoffatom
und
- • = ein cis- oder trans-konfiguriertes Kohlenstoffzentrum darstellen zur Herstellung eines Arzneimittels zur Behandlung von allergischen Erkrankungen.

2. Verwendung nach Anspruch 1 dadurch gekennzeichnet, daß das Macrolacton ausgewählt ist aus der Gruppe

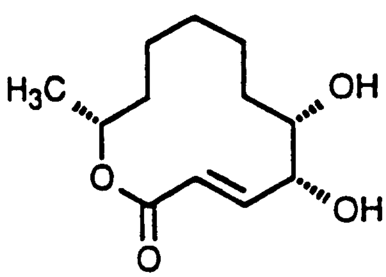

Cladospolid A ,

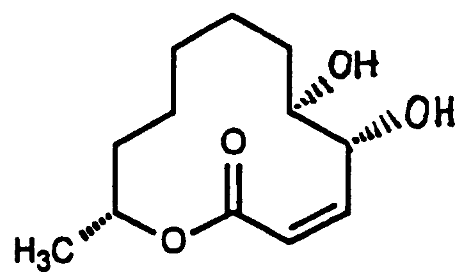

Cladospolid B ,

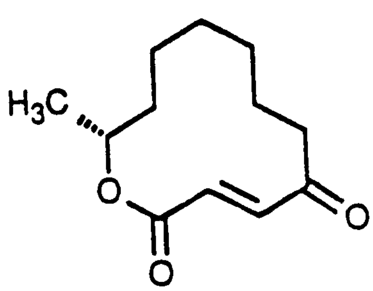

Patulolid A und

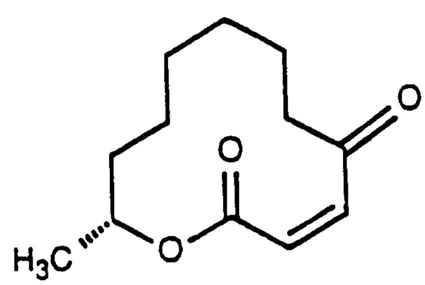

Patulolid B

3. Verwendung nach Ansprüchen 1 und 2, dadurch gekennzeichnet, daß eine Hemmung der Aktivierung proinflammatorisch wirkender Leukozyten bewirkt wird.

4. Verwendung nach Ansprüchen 1, 2 und 3, dadurch gekennzeichnet, daß die allergische Erkrankung eine durch Antigen-induzierte Degranulation peripherer humaner Leukozyten verursachte ist.

**Claims**

1. Use of macrolactones of the general formula I:

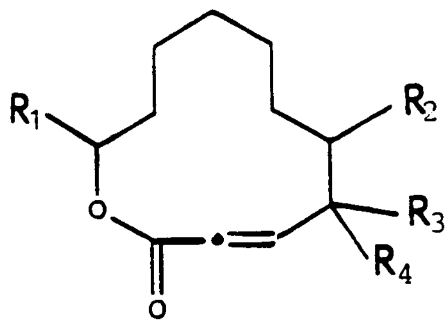

I

in which $R_1$ signifies hydrogen or a $C_1$-$C_4$ alkyl group, $R_2$ represents hydrogen, hydroxyl or an acetoxy group, $R_3$,$R_4$ signify independently from each other hydrogen, hydroxyl or an acetoxy group or represent together an oxygen atom and -• = represents a carbon centre in cis or trans configuration for the preparation of medicaments for the treatment of allergic diseases.

2. Use according to claim 1, characterized in that the macrolactone is selected from the group consisting of

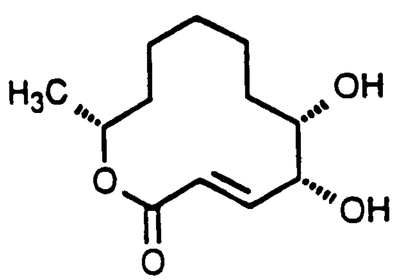

Cladospolid A,

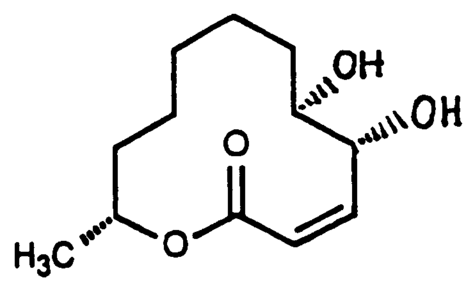

Cladospolid B,

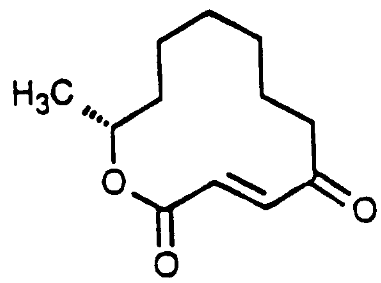

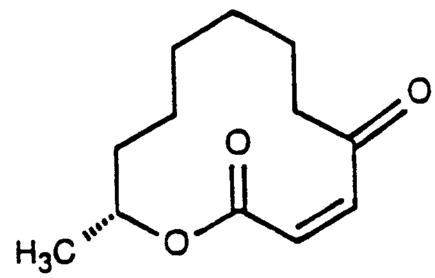

Patulolid A and Patulolid B.

3. Use according to either of claims 1 and 2, characterized in that an inhibition of the activation of proinflammatorily active leucocytes is effected.

4. Use according to any of claims 1, 2 and 3, characterized in that the allergic disease is caused by antigen-induced degranulation of peripheric human leucocytes.

**Revendications**

1. Utilisation de macrolactones de formule générale I

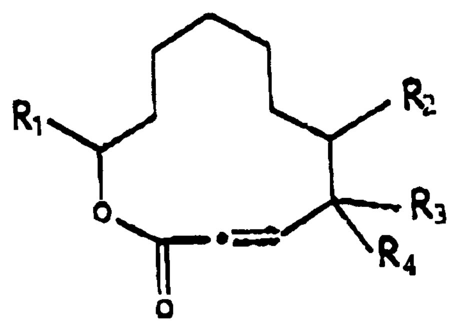

I

dans laquelle $R_1$ représente un atome d'hydrogène ou un reste alkyle en $C_1$ à $C_4$.

$R_2$ un atome d'hydrogène, un reste hydroxyle ou un reste acétoxy.

$R_3$, $R_4$, chacun indépendamment l'un de l'autre, un atome d'hydrogène, un reste hydroxyle ou un reste acétoxy ou ensemble un atome d'oxygène,

et,

- • = représente un centre carboné en configuration cis ou trans pour préparer un médicament pour le traitement des maladies allergiques.

**2.** Utilisation selon la revendication 1, caractérisée en ce que la macrolactone est choisie dans le groupe

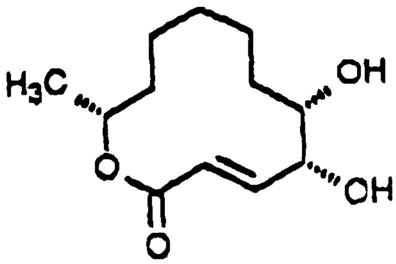

Cladospolide A ,

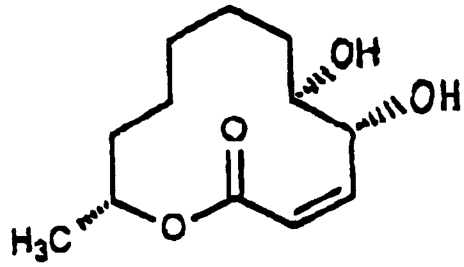

Cladospolide B ,

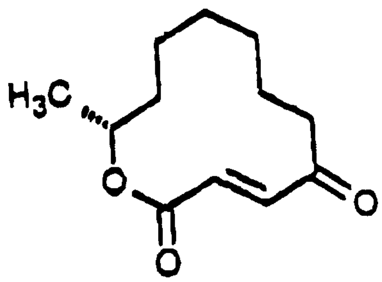

Patulolide A ,

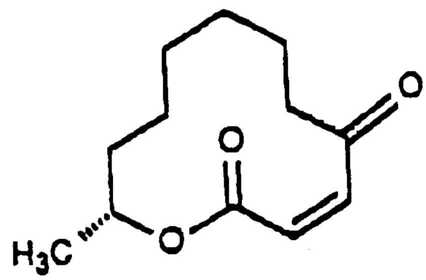

Patulolid B ,

**3.** Utilisation selon les revendications 1 et 2, caractérisée en ce qu'elle produit un ralentissement de l'activation des leucocytes à effet pro-inflammatoire.

**4.** Utilisation selon les revendications 1, 2 et 3, caractérisée en ce que la maladie allergique provoque une dégranulation induite par un antigène des leucocytes périphériques humains.